(19) Europäisches Patentamt
European Patent Office
Office européen des brevets

(11) **EP 4 368 102 A1**

(12) **EUROPEAN PATENT APPLICATION**

(43) Date of publication:
**15.05.2024 Bulletin 2024/20**

(21) Application number: **22206490.9**

(22) Date of filing: **09.11.2022**

(51) International Patent Classification (IPC):
***A61B 5/0531*** (2021.01)      ***A61B 5/0537*** (2021.01)

(52) Cooperative Patent Classification (CPC):
**A61B 5/0531; A61B 5/0537**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **DAMODARAN, Mathivanan**
**5656AG Eindhoven (NL)**

• **PALERO, Jonathan Alambra**
**5656AG Eindhoven (NL)**
• **FRACKOWIAK, Bruno Jean François**
**5656AG Eindhoven (NL)**
• **VARGHESE, Babu**
**5656AG Eindhoven (NL)**

(74) Representative: **Philips Intellectual Property & Standards**
**High Tech Campus 52**
**5656 AG Eindhoven (NL)**

(54) **DETERMINING A HYDRATION LEVEL IN SKIN OF A SUBJECT**

(57)      According to an aspect, there is provided an apparatus (100) for determining a hydration level in skin of a subject. The apparatus (100) comprises: a first electrode (102) arranged to contact the skin of the subject; a second electrode (104) arranged to contact the skin of the subject; a radiofrequency, RF, generator unit (106) configured to supply an RF voltage between the first electrode and the second electrode at each of a plurality of distinct frequencies such that a current is able to flow from the first electrode, via the skin of the subject, to the second electrode; a skin impedance measurement unit (108) configured to measure an impedance of the skin between the first electrode and the second electrode at each of the plurality of distinct frequencies; and a processing unit (110) in operative communication with the RF generator unit and the skin impedance measurement unit, the processing unit configured to: determine, based on a gradient of a difference in the impedance of the skin at each of the plurality of distinct frequencies, a hydration level of the skin.

**EP 4 368 102 A1**

Fig. 1

**Description**

FIELD OF THE INVENTION

The invention relates to an apparatus and method for determining a hydration level in skin of a subject and, more particularly, to determining a hydration level in skin of a subject based on impedance data.

BACKGROUND OF THE INVENTION

The safety and efficacy associated with a personal care device, such as a skin treatment device, may depend on a condition of the skin, such as whether the skin is wet or dry. Therefore, interactions between a personal care device and the skin may be dependent on a condition of the skin. For example, radiofrequency energy may be used to provide a skin warming experience during use of a personal care device, such as during shaving. A level of radiofrequency energy delivered to the skin may depend on a range of factors, such as factors relating to skin condition, which may need to be accounted for when determining settings of radiofrequency energy generation to maintain the safety of a subject in which the personal care device is being applied.

The present invention aims to address the treatment safety and efficacy issues associated with wet and dry conditions of the skin.

SUMMARY OF THE INVENTION

A hydration level of skin of a subject may affect parameters relating to skin surface friction, optical coupling efficiency, electrical contact and effective impedance. It is an aim of the invention described herein to provide a way in which a hydration level in skin of a subject can be determined such that interactions between a personal care device, such as a skin treatment device, and the skin of a subject may be adjusted dependent on the skin hydration, thereby accounting for parameters relating to the skin.

According to a first specific aspect, there is provided an apparatus for determining a hydration level in skin of a subject. The apparatus comprises: a first electrode arranged to contact the skin of the subject; a second electrode arranged to contact the skin of the subject; a radiofrequency, RF, generator unit configured to supply an RF voltage between the first electrode and the second electrode at each of a plurality of distinct frequencies such that a current is able to flow from the first electrode, via the skin of the subject, to the second electrode; a skin impedance measurement unit configured to measure an impedance of the skin between the first electrode and the second electrode at each of the plurality of distinct frequencies; and a processing unit in operative communication with the RF generator unit and the skin impedance measurement unit. The processing unit is configured to: determine, based on a gradient of a difference in the impedance of the skin at each of the plurality of distinct frequencies, a hydration level of the skin.

In some embodiments, the processing unit may be further configured to: generate, based on the hydration level of the skin, an instruction signal for delivery to a recipient device.

The instruction signal may, in some embodiments, comprise a signal to adjust the RF voltage supplied between the first electrode and the second electrode.

In some embodiments, each of the plurality of distinct frequencies may be in the frequency range 0.5 MHz to 100 MHz.

Each of the plurality of distinct frequencies may, in some embodiments, be in the frequency range 1 MHz to 10 MHz.

In some embodiments, the RF voltage may comprise a value in the range 5 V to 30 V.

According to a second aspect, there is provided a personal care device comprising an apparatus as described herein.

In some embodiments, the processing unit may be further configured to: generate, based on the hydration level of the skin, a control signal to control an operating parameter of the personal care device.

The personal care device may further comprise a motor. The operating parameter may comprise a parameter of the motor.

In some embodiments, the personal care device may further comprise an IR light source configured to heat the skin of the subject. The operating parameter may comprise a parameter relating to an IR light intensity of the IR light source.

The personal care device may further comprise a display element. The operating parameter may comprise a parameter of the display element.

According to a third aspect, a computer-implemented method for determining a hydration level in skin of a subject is provided. The method comprises: operating a radiofrequency, RF, generator to generate an RF voltage to be delivered between a first electrode and a second electrode , at each of a plurality of distinct frequencies such that a current is able to flow from the first electrode, via the skin of the subject, to the second electrode when the first and second electrodes are in contact with the skin; determining an impedance of the skin between the first electrode and the second electrode at each of the plurality of distinct frequencies; and generating, based on a gradient of a difference in the impedance of the skin at each of the plurality of distinct frequencies, a hydration level of the skin.

In some embodiments, the method may further comprise generating, based on the hydration level of the skin, an instruction signal for delivery to a recipient device.

The instruction signal may, in some embodiments, comprise a signal to adjust the RF voltage supplied between the first electrode and the second electrode.

According to a fourth aspect, a computer program product is provided, the computer program product comprises a non-transitory computer readable medium, the computer readable medium having computer readable code embodied therein, the computer readable code being configured such that, on execution by a suitable computer or processor, the computer or processor is caused to perform steps of the method described herein.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1 is a schematic illustration of an example of an apparatus for determining a hydration level in skin of a subject;
Fig. 2 is a schematic illustration of an example of an arrangement of electrodes;
Fig. 3 is a plot representing an example of how impedance may vary with frequency for dry skin and wet skin;
Fig. 4 is a plot representing a further example of how impedance varies with frequency for dry skin and wet skin;
Fig. 5 is a schematic illustration of an example of a personal care device;
Fig. 6 is a schematic illustration of a further example of an arrangement of electrodes;
Fig. 7 is a flowchart of an example of a method for determining a hydration level in skin of a subject; and
Fig. 8 is a schematic illustration of a processor in communication with a non-transitory computer readable medium.

DETAILED DESCRIPTION OF THE EMBODIMENTS

A level of hydration of skin of a subject may vary over time, may be different for different regions of skin of the subject, and may be different to another subject's level of skin hydration. Determining a level of hydration of skin of a subject is useful for a number of reasons, such as setting an operating parameter of a personal care device in accordance with a determined level of skin hydration. In particular, a priori knowledge of a level of hydration of skin of a subject can be important in terms of controlling an operating parameter of a device, such as a personal care device, to maintain safety of a subject in which the device is being applied. For instance, radiofrequency energy may be applied to the skin of a subject to provide a warming effect within the skin. However, operating parameters associated with radiofrequency generation may need to be set, or adjusted, based on a level of hydration of the skin, to avoid the potential formation of hot spots and/or burning.

Electrical properties associated with the skin of a subject may vary significantly with the condition of the skin, such as whether the skin is wet or dry, and thus measures of electrical properties associated with the skin may be used to determine a level of hydration of the skin. For example, the skin may have relatively low electrical impedance while dry skin may have relatively high electrical impedance. Electrical impedance may be different for different areas of skin of the body (e.g., face, hands, and the like), and may vary for different people and skin anatomies. However, a determination of electrical impedance may not be a robust and reliable measure of skin hydration due to the factors affecting skin hydration mentioned previously. Therefore, it is an objective of the present invention to provide a robust and reliable way in which a hydration level of the skin of a subject can be determined based on electrical properties of the skin. More particularly, it is a further objective of the present invention to provide an operating parameter of an RF generator unit in a personal care device such that RF energy is delivered in a safe way (e.g., such that a pleasant warming experience is provided to a subject in which the personal care device is being applied, such that the occurrence of hot spots and/or burning is prevented, or the like).

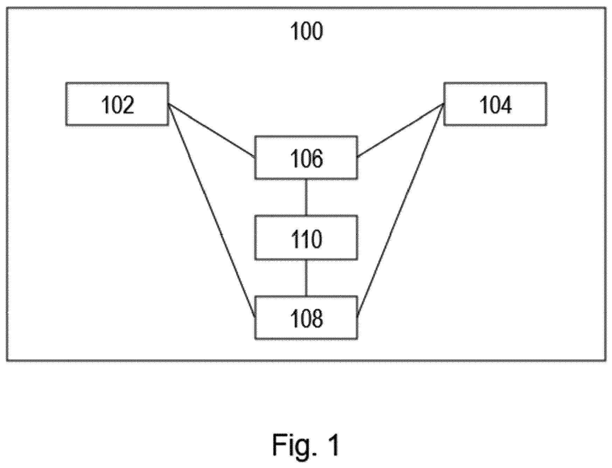

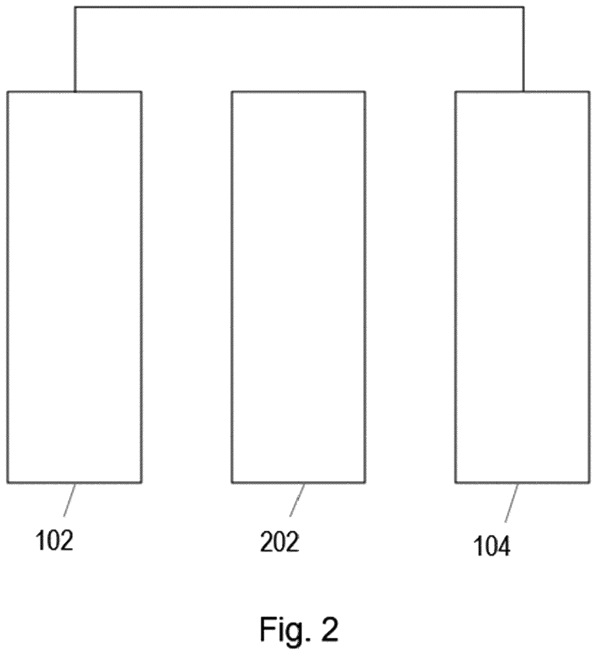

According to a first aspect, the present invention provides an apparatus 100 for determining a hydration level in skin of a subject. Fig. 1 shows an example of such an apparatus 100. The apparatus 100 comprises a first electrode 102 arranged to contact the skin of the subject and a second electrode 104 arranged to contact the skin of the subject. The electrodes 102, 104 may be referred to as probe, or sensing, RF skin contacting electrodes. In some examples, the first electrode 102 and the second electrode 104 may be connected (e.g., electrically connected). The first electrode 102 and the second electrode 104 may be connected (e.g., connected via a radiofrequency generator unit (described below)). In some examples, the apparatus 100 may comprise three or more electrodes. For example, the apparatus 100 may comprise the first electrode 102, the second electrode 104 and a third electrode (not shown in Fig. 1). The third electrode may be a grounded electrode and/or may be located between the first electrode 102 and the second electrode 104. Fig. 2 shows an example of a schematic view of an RF electrode configuration comprising the first electrode 102, the second electrode 104 and a third electrode 202. The first electrode 102 may be connected to the third electrode 202 and/or the second electrode 104 may be connected to the third electrode 202. In some examples, the first electrode 102 may be connected to the second electrode 104, and the first electrode 102 may be connected to the third electrode 202 and/or the second electrode 104 may be connected to the third electrode 202. The electrodes may have the same, or different, dimensions. For example, the first electrode 104 and the second electrode may each have dimensions of 25 mm × 4 mm, and the third electrode 202 may have dimensions 25 mm × 5 mm. The electrodes may be separated by the same, or different, amounts. For example, the first electrode 102 may be separated from the third electrode 202 by 4 mm, and the second electrode 104 may be separated from the third electrode 202 by 4 mm. In some examples, a dimension (e.g., width) or the shortest side of an electrode may comprise a value in the range 0.1 mm to 10 mm. In some examples, electrodes may comprise a rectangular shape, a square shape, a circular shape, or the like.

The apparatus 100 further comprises a radiofrequency, RF, generator unit 106 configured to supply an RF voltage between the first electrode 102 and the second electrode 104 at each of a plurality of distinct frequencies such that a current is able to flow from the first electrode, via the skin of the subject, to the second electrode. The RF generator unit 106 may comprise an RF energy source configured to generate RF energy. The RF generator unit 106 may be referred to as a probe RF generator unit for delivering probe RF energy to the probe RF electrodes. In other words, the RF generator unit 106 may generate RF energy for delivery to the skin of a subject via the first electrode 102 and the second electrode 104, for example to increase the temperature of the subject's skin by around 1 to 4 °C. The RF voltage may comprise a voltage in the range 5 V to 30 V, 0 V to 30 V, 5 V to 50 V, or the like. The voltage signals may comprise a frequency in the range 0.5 MHz to 100 MHz, 1 MHz to 10 MHz, or the like. In other words, the plurality of distinct frequencies may comprise a frequency in the range 0.5 MHz to 100 MHz, 1 MHz to 10 MHz, or the like. In some examples, the RF generator unit 106 may be configured to supply the RF voltage between the first electrode 102 and the second electrode 104 in a pulsed manner, for example with each pulse having a pulse duration in the range 10 ms to 100 ms, or the like. A plurality of distinct frequencies refers to two or more different frequencies (e.g., 10 MHz and 50 MHz). In some examples, relatively high voltages may be applied to the electrodes when using relatively small electrodes (e.g., < 1 mm).

In other words, the RF generator unit 106 is configured to supply a voltage between the first electrode 102 and the second electrode 104 such that an electric field may be generated, or caused to extend, between the first electrode 102 and the second electrode 104. When the first electrode 102 and the second electrode 104 are in contact with skin of the subject (e.g., when the apparatus 100 is in use), skin acts as a capacitor, storing electrical charge, due to the polarization of the macromolecules (e.g., proteins or cell elements) with respect to the electrical field. When the voltage of the electrodes is switched (e.g., the first electrode 102 may change from positive 10 V to negative 10 V and the second electrode 104 may change from negative 10 V to positive 10 V), the polarization changes and releases those electrical charges, thus leading to an electrical current. This dielectric coupling of the skin lowers the impedance for alternating RF currents, whereas the skin acts as an insulator for DC current, with higher impedance values. Additionally, polarization change of the skin macromolecules leads to dielectric losses, and thus dielectric heating. Therefore, when the voltage of the electrodes is alternated at a high frequency (e.g., radiofrequency), then more significant heating may be realised in the skin of a subject.

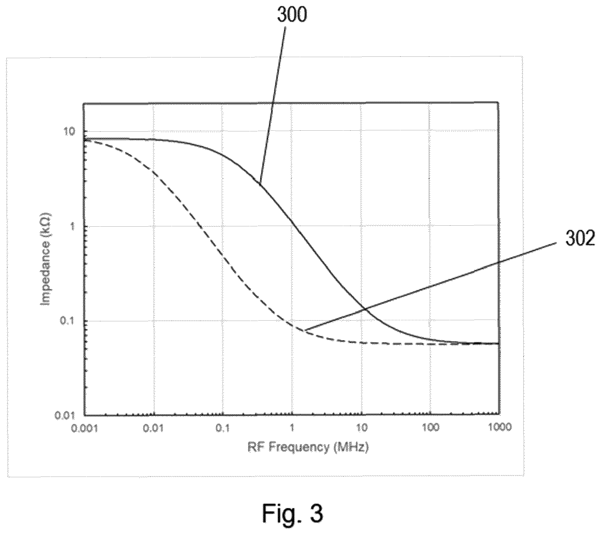

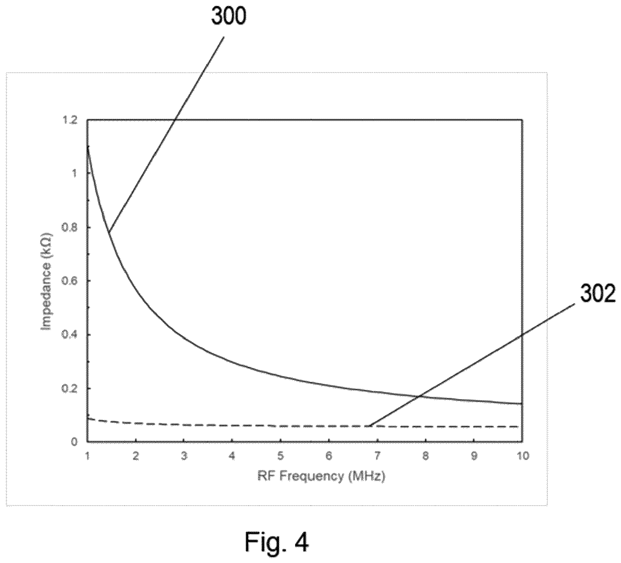

Skin hydration may depend on the ability of skin to bind water with macromolecules of the skin (e.g., the ability to bind water with keratinized tissues of the skin), which may lead to changes in the skin dielectric properties. Additionally, the skin may comprise water that is not bound to molecules of the skin (e.g., bulk water), which may contribute to an ionic conductivity of the skin. Thus, wet skin may be more conductive than dry skin. Those changes in the skin electrical properties may increase conductive and dielectric heating. Determining a level of skin hydration can therefore be important, for example, in applications applying RF energy, such that localised heating and/or burning of the skin may be avoided. The apparatus 100 further comprises a skin impedance measurement unit 108 configured to measure an impedance of the skin between the first electrode 102 and the second electrode 104 at each of the plurality of distinct frequencies. Impedance of the skin may be dependent on skin hydration, as explained previously, and may be determined based on a measurement of current and voltage. Fig. 3 is a plot representing an example of how impedance may vary with frequency for dry skin (represented by line 300) and wet skin (represented by line 302) over the frequency range 0.001 MHz to 1000 MHz, when the first electrode 102 and the second electrode 104 of the apparatus 100 are placed on the skin of a subject. Fig. 4 is a plot representing an example of how impedance may vary with frequency for dry skin (line 300) and wet skin (line 302) over the frequency range 1 MHz to 10 MHz using the same data as used in Fig. 3. In the examples shown in Figs. 3 and 4, resistive behaviour of the current in the stratum corneum (i.e., the surface of the skin) dominates at low frequencies (e.g., < 0.1 MHz) resulting in a plateau of high impedance. In the examples shown in Figs. 3 and 4, capacitive coupling across the stratum corneum is maximal at high frequencies (e.g., > 100 MHz) resulting in a higher electrical current passing through towards the dermis and thus the resistive behaviour of the current in the dermis dominates leading to a plateau of low impedance. In other words, at low frequencies, the stratum corneum has high impedance (e.g., is surface of the skin is highly resistive to current) and, as the frequency is increased, the impedance reduces (e.g., the surface of the skin is less resistive to current), and the subsurface regions of the skin dominate in terms of resistance. In the examples in Figs. 3 and 4, the capacitive coupling gradually increases for frequencies in an intermediary range (e.g., between 0.1 MHz and 100 MHz), which may be referred to as a transition regime. When the skin is wet, the skin hydrates, which may result in a higher capacitive coupling, which, in turn, may shift the transition of high impedance to low impedance towards lower frequencies. Therefore, in this example, frequencies in the range 1 MHz to 10 MHz may be closer to a flat plateau of low impedance, making the impedance appear relatively constant in wet conditions for this frequency range.

The apparatus 100 further comprises a processing unit 110 which may be in operative communication with the RF generator unit 106 and the skin impedance measurement unit 108. The processing unit 110 is configured to determine, based on a gradient of a difference in the impedance of the skin at each of the plurality of distinct frequencies, a hydration level of the skin. For example, a gradient of a difference in the impedance of the skin at each of two distinct frequencies may be determined using the equation:

$$\frac{Z_2 - Z_1}{f_2 - f_1},$$

where $Z_1$ is an impedance of the skin at frequency $f_1$ and $Z_2$ is an impedance of the skin at frequency $f_2$. In other words, a slope of impedance as a function of frequency is determined. The gradient, or slope, of impedance with frequency may be determined using a slope fitting algorithm, such as a least squared fitting routine, or the like. In some examples, skin may be classified according to Table 1:

Table 1

| Skin state classification | Water content | Impedance gradient ($\times$ -$\Omega$/MHz) |
|---|---|---|
| **Very dry skin** | < 30 % | > 300 |
| **Dry skin** | 30 % to 40 % | 200 to 300 |
| **Wet skin** | 40 % to 50 % | 10 to 200 |
| **Very wet skin** | > 50 % | < 10 |

In some examples, the apparatus 100 may comprise a memory for storing impedance data, skin hydration data, or the like. The apparatus 100 may comprise a transmitter configured to transmit data (e.g., impedance data, skin hydration data, or the like) to a memory external to the device (e.g., a server located in the cloud, or the like). In some examples, a processor located external to the apparatus 100 (e.g., an external processor, a processor located in the cloud, or the like) may be configured to determine, based on a gradient of a difference in the impedance of the skin at each of the plurality of distinct frequencies, a hydration level of the skin. A processor located external to the apparatus 100 may be configured to receive data from a transmitter associated with the apparatus 100, from a memory external to the apparatus 100, or the like.

The RF energy applied to the skin of the subject may cause a small amount of heating in the skin of the subject. However, operating parameters of the apparatus 100 (e.g., voltage applied to the electrodes, a duration in which the RF energy is applied to the skin of a subject, and the like) may be set such that hot spots in, and/or burning of, the skin is avoided. For example, applying the RF energy to the skin of the subject for 1 second may be sufficient to obtain enough data (e.g., impedance data) such that a determination of a level of hydration of the skin of a subject can be determined while avoiding excessive heat generation in the skin. It may be beneficial to reach a balance between minimizing the skin heating effect due to the applied RF energy and the maximizing the current flow to minimize calculation error. Higher voltages may be preferred for a high skin impedance situation, such as when using very small electrodes (e.g., electrodes having a width of less than 1 mm). In such examples, to minimize heating, pulses of RF energy may be used, having a pulse duration in the range 10 ms and 100 ms. In some examples, each electrode may have a width of between 0.1 mm and 10 mm, an RF voltage of between 5 V and 30 V may be used, with an RF frequency of between 1 MHz and 10 MHz.

In some embodiments, the processing unit 110 may be further configured to generate, based on the hydration level of the skin, an instruction signal for delivery to a recipient device. A recipient device may be a personal care device (e.g., a personal care device comprising the apparatus 100). In some examples, the recipient device may be a device external (e.g., separate) to the apparatus 100 (e.g., an interactive mirror, a smart phone, a server, a wearable device or the like). The instruction signal may comprise a control signal (e.g., a control signal to control an operating parameter of the apparatus 100), a signal to cause display of an element in an interactive mirror, or the like.

In some embodiments, the instruction signal may comprise a signal to adjust the RF voltage supplied between the first electrode and the second electrode.

For example, higher frequencies (e.g., higher RF frequencies) may lead to, or be associated with, a lower skin impedance. A lower skin impedance may lead to a relatively large heating effect (e.g., RF heating) compared to a lower frequency (e.g., due to a relatively large current flow between the electrodes for relatively high frequencies compared to relatively low frequencies). The current flow between the electrodes may depend on the voltage applied to the electrodes (e.g., a current between the electrodes may be higher for a larger voltage difference, or potential difference, between the electrodes). Relatively low voltages (e.g., RF voltages) may be applied to the electrodes for relatively high frequencies to minimise heat generation in the skin. In some examples, increasing a current flow between the electrodes may improve an accuracy of a determination of impedance of the skin between the electrodes. For example, the RF voltage may be increased, to increase current flow, and/or the effective resistance of the system may be reduced, for example by using larger electrodes. Increasing a current flow between the electrodes may lead to a relatively larger heating effect (e.g., compared to a relatively low current flow between the electrodes). Voltages applied to the electrodes may therefore depend on the frequency with which voltages are applied to the electrodes (e.g., a voltage and/or a frequency may be chosen based on a minimum desired accuracy level of impedance). In some examples, a first RF voltage may be supplied between the first electrode 102 and the second electrode 104 at a first frequency, and a second RF voltage may be supplied between the first electrode 102 and the second electrode 104 at a second frequency. For example, the first RF voltage may be 10 V and may have a first frequency of 1 MHz, and the second RF voltage may be 5 V and may have a second frequency of 10 MHz.

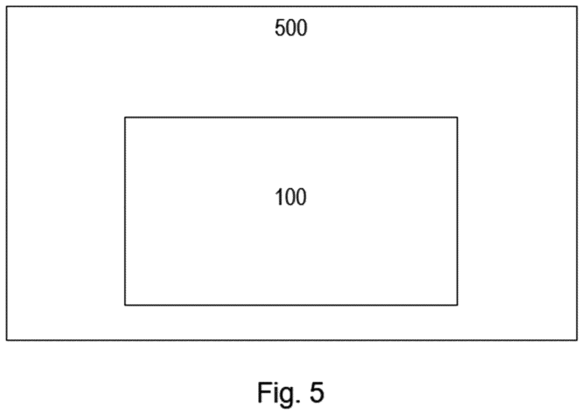

According to a second aspect, there is provided a personal care device 500 comprising an apparatus 100 as described herein. Fig. 5 shows an example of a personal care device 500 comprising an apparatus 100. A personal care device 500 may comprise a hair cutting device (e.g., a shaving device, an electric shaver, a beard trimmer, a hair trimmer, or the like), a skin care device (e.g., a skin firming device, a skin rejuvenation device, a skin cleansing device, or the like), or the like. A skin rejuvenation device may comprise a component configured to supply, or apply, radiofrequency energy to the skin. In some examples, a skin rejuvenation device may comprise a microdermabrasion device, or the like. A skin cleansing device may comprise a mechanical rotating brush.

In some embodiments, the processing unit 110 may be configured to generate, based on the hydration level of the skin, a control signal to control an operating parameter of the personal care device.

In some embodiments, the operating parameter may comprise an operating parameter of the RF generator unit 106 (e.g., a voltage supplied between the first electrode 102 and the second electrode 104, a duration in which an alternating voltage is applied to the electrodes (e.g., 1 second), or the like). Adjusting an operating parameter of the RF generator unit based on a determined level of skin hydration may lead to an adjustment in a level of heat generated in the skin, skin temperature, warming depth within the skin, warming rate of the skin, or the like. For example, lower voltages, or a lower potential difference, may be supplied between the electrodes for relatively wet, or hydrated, skin because wet skin may be associated with a lower impedance such that RF heating is more efficient. An operating parameter of the RF generator unit 106 may therefore be adjusted for safety reasons.

In some embodiments, the personal care device may comprise a motor. The operating parameter may comprise a parameter of the motor (e.g., a speed of the motor, a current supplied to the motor, a voltage supplied to the motor, or the like). Skin hydration may affect skin surface friction, such that it may be desirable to alter an operating parameter of a motor of a personal care device (e.g., a speed of a cutting element of a hair cutting device). In some examples, a lower motor current of a cutting element of a hair cutting device may be required, or desired, because wet hair may be softer and easier to cut. Skin hydration may be indicative of how wet hair is, and thus a motor current may be adjusted based on the skin hydration accordingly.

In some embodiments, the personal care device may comprise an infrared, IR, light source configured to heat the skin of the subject. The operating parameter may comprise a parameter relating to an IR light intensity of the IR light source. Skin hydration may affect an optical coupling efficiency of light (e.g., IR light) into the skin, a level of transmittance of light between a light source and the skin, a level of scattering of light between a light source and the skin, or the like. IR light may be used to provide a skin warming effect. Relatively wet, or hydrated, skin may be associated with better optical coupling efficiency. Therefore, for reasons of safety, a lower light intensity may be used for relatively wet skin.

In some embodiments, the personal care device may comprise a display element. The operating parameter may comprise a parameter of the display element. For example, a display element may comprise a light on a personal care device indicating whether it is safe to use the device. For example, a red light may indicate that the device is not safe to use (e.g., could result in burning), whereas a green light may indicate that the device is safe to use.

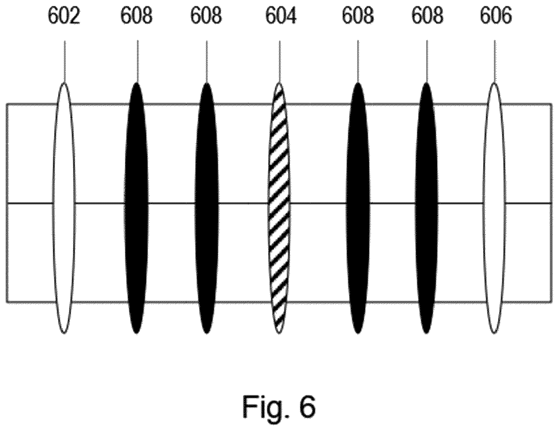

In some examples, the personal care device 500 may comprise a hair cutting device (e.g., an electrical beard trimmer) comprising a bi-directional beard trimmer unit mounted on a handheld housing unit and an RF-delivering comb attachment comprising bi-directional guiding comb teeth and RF electrodes. An RF generator unit 106 may be mounted in the handheld housing unit. During use, the RF generator unit 106 may provide RF energy to the skin via RF electrodes (e.g., the first electrode 102 and the second electrode 104) at two different RF frequencies (e.g., 1 MHz and 5 MHz) for which impedance is measured and recorded. After a pre-set probing time (e.g., 1 second for each frequency), the recorded impedance data is used to determine the gradient, or slope, of impedance with frequency. If the gradient has a magnitude of approximately -150 $\Omega$/MHz (e.g., 140 $\Omega$/MHz to 160 $\Omega$/MHz, or the like), the conditions may be deemed to be dry. If the gradient has a magnitude of approximately -5 $\Omega$/MHz, the conditions may be deemed to be wet. Fig. 6 is a schematic illustration of a further example of an arrangement of electrodes. A hair cutting device may comprise the arrangement of electrodes shown in Fig. 6.

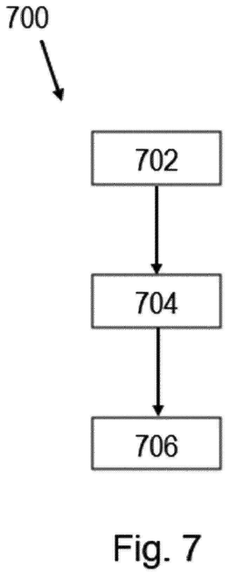

According to a third aspect, there is provided a method (e.g., a computer-implemented method) for determining a hydration level in skin of a subject. Fig. 7 is a flowchart of an example of a computer-implemented method 700 for determining a hydration level in skin of a subject. In some examples, a processor may be configured to perform one or more steps of the method 700. The method 700 comprises, at step 702, operating a radiofrequency, RF, generator to generate an RF voltage to be delivered between a first electrode and a second electrode , at each of a plurality of distinct frequencies such that a current is able to flow from the first electrode, via the skin of the subject, to the second electrode when the first and second electrodes are in contact with the skin.

The method 700 comprises, at step 704, determining an impedance of the skin between the first electrode and the second electrode at each of the plurality of distinct frequencies.

The method 700 comprises, at step 706, generating, based on a gradient of a difference in the impedance of the skin at each of the plurality of distinct frequencies, a hydration level of the skin.

In some embodiments, the method 700 may comprise generating, based on the hydration level of the skin, an instruction signal for delivery to a recipient device.

In some embodiments, the instruction signal may comprise a signal to adjust the RF voltage supplied between the first electrode and the second electrode.

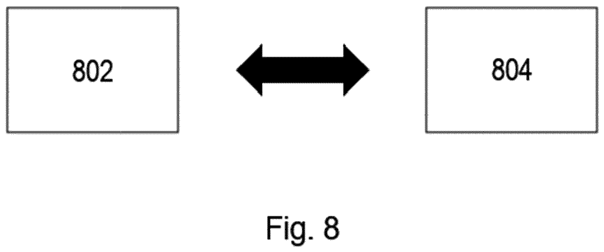

According to a fourth aspect, there is provided a computer program product. Fig. 8 is a schematic illustration of an example of a processor 802 in communication with a computer-readable medium 804. The computer program product comprises a non-transitory computer readable medium 804, the computer readable medium having computer readable code embodied therein, the computer readable code being configured such that, on execution by a suitable computer or processor 802, the computer or processor is caused to perform steps of the methods 700 described herein.

The processor 110, 802 can comprise one or more processors, processing units, multicore processors or modules that are configured or programmed to control the apparatus 100 in the manner described herein. In particular implementations, the processor 110, 802 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is

# EP 4 368 102 A1

embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1.  An apparatus (100) for determining a hydration level in skin of a subject, the apparatus comprising:

    a first electrode (102) arranged to contact the skin of the subject;
    a second electrode (104) arranged to contact the skin of the subject;
    a radiofrequency, RF, generator unit (106) configured to supply an RF voltage between the first electrode and the second electrode at each of a plurality of distinct frequencies such that a current is able to flow from the first electrode, via the skin of the subject, to the second electrode;
    a skin impedance measurement unit (108) configured to measure an impedance of the skin between the first electrode and the second electrode at each of the plurality of distinct frequencies; and
    a processing unit (110) in operative communication with the RF generator unit and the skin impedance measurement unit, the processing unit configured to:
    determine, based on a gradient of a difference in the impedance of the skin at each of the plurality of distinct frequencies, a hydration level of the skin.

2.  An apparatus (100) according to claim 1, wherein the processing unit is further configured to:
    generate, based on the hydration level of the skin, an instruction signal for delivery to a recipient device.

3.  An apparatus (100) according to claim 2, wherein the instruction signal comprises a signal to adjust the RF voltage supplied between the first electrode and the second electrode.

4.  An apparatus (100) according to any preceding claim, wherein each of the plurality of distinct frequencies is in the frequency range 0.5 MHz to 100 MHz.

5.  An apparatus (100) according to any of claims 1 to 3, wherein each of the plurality of distinct frequencies is in the frequency range 1 MHz to 10 MHz.

6.  An apparatus (100) according to any preceding claim, wherein the RF voltage comprises a value in the range 5 V to 30 V.

7.  A personal care device (500) comprising an apparatus (100) according to any of claims 1 to 6.

8.  A personal care device (500) according to claim 7, wherein the processing unit (110) is further configured to:
    generate, based on the hydration level of the skin, a control signal to control an operating parameter of the personal care device.

9.  A personal care (500) device according to claim 8, wherein the personal care device further comprises a motor, and wherein the operating parameter comprises a parameter of the motor.

10. A personal care device (500) according to claim 8, wherein the personal care device further comprises an IR light source configured to heat the skin of the subject, and wherein the operating parameter comprises a parameter relating to an IR light intensity of the IR light source.

**11.** A personal care device (500) according to claim 8, wherein the personal care device further comprises a display element, and wherein the operating parameter comprises a parameter of the display element.

**12.** A computer-implemented method (700) for determining a hydration level in skin of a subject, the method comprising:

operating (702) a radiofrequency, RF, generator to generate an RF voltage to be delivered between a first electrode and a second electrode, at each of a plurality of distinct frequencies such that a current is able to flow from the first electrode, via the skin of the subject, to the second electrode when the first and second electrodes are in contact with the skin;

determining (704) an impedance of the skin between the first electrode and the second electrode at each of the plurality of distinct frequencies; and

generating (706), based on a gradient of a difference in the impedance of the skin at each of the plurality of distinct frequencies, a hydration level of the skin.

**13.** A method (700) according to claim 12, further comprising:
generating, based on the hydration level of the skin, an instruction signal for delivery to a recipient device.

**14.** A method (700) according to claim 13, wherein the instruction signal comprises a signal to adjust the RF voltage supplied between the first electrode and the second electrode.

**15.** A computer program product comprising a non-transitory computer readable medium (804), the computer readable medium having computer readable code embodied therein, the computer readable code being configured such that, on execution by a suitable computer or processor (802), the computer or processor is caused to perform the method (700) according to any of claims 12 to 14.

Fig. 1

Fig. 2

Fig. 3

300

302

Fig. 4

Fig. 5

602     608     608     604     608     608     606

Fig. 6

700

702

704

706

Fig. 7

802 ⟷ 804

Fig. 8

**EP 4 368 102 A1**

Europäisches
Patentamt
European
Patent Office
Office européen
des brevets

# EUROPEAN SEARCH REPORT

Application Number

EP 22 20 6490

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/345263 A1 (VARGHESE BABU [NL] ET AL) 5 November 2020 (2020-11-05) | 1,2, 4-13,15 | INV. A61B5/0531 A61B5/0537 |
| Y | * paragraphs [0019], [0057] – [0061], [0072] – [0075], [0079] – [0080]; claims 16,23; figures 2,3 * | 3,14 | |
| X | US 2020/345294 A1 (VARGHESE BABU [NL] ET AL) 5 November 2020 (2020-11-05) | 1,2, 4-13,15 | |
| Y | * paragraph [0070]; figure 2 * | 3,14 | |
| Y | US 2018/103891 A1 (MOON JONG SOO [KR] ET AL) 19 April 2018 (2018-04-19) * paragraph [0049] * | 3,14 | |
| X | US 2022/240806 A1 (ZHU FANSAN [US] ET AL) 4 August 2022 (2022-08-04) * paragraphs [0007], [0036], [0051] – [0073]; claim 7; figure 1 * | 1,12 | |
| X | WO 2022/069550 A1 (MODE SENSORS AS [NO]) 7 April 2022 (2022-04-07) * paragraphs [0038] – [0079]; figures 1,8,8b * | 1,12 | TECHNICAL FIELDS SEARCHED (IPC) A61B |
| A | US 2008/133184 A1 (CHEN ZHIHAN [US]) 5 June 2008 (2008-06-05) * figures 2,6 * | 1,12 | |

The present search report has been drawn up for all claims

| Place of search | Date of completion of the search | Examiner |
|---|---|---|
| Berlin | 5 April 2023 | Kronberger, Raphael |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
.......................................................................................................
& : member of the same patent family, corresponding
    document

EPO FORM 1503 03.82 (P04C01)

18

## ANNEX TO THE EUROPEAN SEARCH REPORT
## ON EUROPEAN PATENT APPLICATION NO.

EP 22 20 6490

This annex lists the patent family members relating to the patent documents cited in the above-mentioned European search report.
The members are as contained in the European Patent Office EDP file on
The European Patent Office is in no way liable for these particulars which are merely given for the purpose of information.

05-04-2023

| Patent document cited in search report | | | Publication date | Patent family member(s) | | | Publication date |
|---|---|---|---|---|---|---|---|
| US 2020345263 | A1 | | 05-11-2020 | CN | 112423647 | A | 26-02-2021 |
| | | | | EP | 3505045 | A1 | 03-07-2019 |
| | | | | EP | 3731738 | A1 | 04-11-2020 |
| | | | | JP | 6928181 | B2 | 01-09-2021 |
| | | | | JP | 2021506524 | A | 22-02-2021 |
| | | | | US | 2020345263 | A1 | 05-11-2020 |
| | | | | WO | 2019129812 | A1 | 04-07-2019 |
| US 2020345294 | A1 | | 05-11-2020 | CN | 111556726 | A | 18-08-2020 |
| | | | | EP | 3505046 | A1 | 03-07-2019 |
| | | | | EP | 3731734 | A1 | 04-11-2020 |
| | | | | JP | 7212690 | B2 | 25-01-2023 |
| | | | | JP | 2021508527 | A | 11-03-2021 |
| | | | | US | 2020345294 | A1 | 05-11-2020 |
| | | | | WO | 2019129597 | A1 | 04-07-2019 |
| US 2018103891 | A1 | | 19-04-2018 | KR | 101661902 | B1 | 04-10-2016 |
| | | | | US | 2018103891 | A1 | 19-04-2018 |
| | | | | WO | 2016208932 | A1 | 29-12-2016 |
| US 2022240806 | A1 | | 04-08-2022 | CA | 3120465 | A1 | 25-06-2020 |
| | | | | CN | 113316417 | A | 27-08-2021 |
| | | | | EP | 3870043 | A1 | 01-09-2021 |
| | | | | KR | 20210105945 | A | 27-08-2021 |
| | | | | US | 2020196903 | A1 | 25-06-2020 |
| | | | | US | 2022240806 | A1 | 04-08-2022 |
| | | | | WO | 2020131562 | A1 | 25-06-2020 |
| WO 2022069550 | A1 | | 07-04-2022 | NO | 20201071 | A1 | 31-03-2022 |
| | | | | WO | 2022069550 | A1 | 07-04-2022 |
| US 2008133184 | A1 | | 05-06-2008 | NONE | | | |

EPO FORM P0459

For more details about this annex : see Official Journal of the European Patent Office, No. 12/82